# EUROPÄISCHE PATENTSCHRIFT

(11) **EP 1 226 838 B1**
(45) Veröffentlichungstag und Bekanntmachung des Hinweises auf die Patenterteilung: **09.11.2016**
(21) Anmeldenummer: 02001475.9
(22) Anmeldetag: 22.01.2002
(51) Int. Cl.: A61M 1/16, A61M 1/36, A61M 5/168

(54) **Therapieeinrichtung**
Therapy device
Dispositif pour thérapie

(30) Priorität: 24.01.2001 DE 10103048
(43) Veröffentlichungstag der Anmeldung: 31.07.2002
(73) Patentinhaber: B. Braun Avitum AG, 34212 Melsungen (DE)
(72) Erfinder: Röher, Otfried, 01139 Dresden (DE)
(74) Vertreter: Winter, Brandl, Fürniss, Hübner, Röss, Kaiser, Polte - Partnerschaft mbB

(56) Entgegenhaltungen:
- EP-A- 0 956 872
- US-A- 4 468 219
- US-A- 5 178 603

## Beschreibung

Die Erfindung betrifft eine Therapieeinrichtung mit einem Blutdruckmeßgerät und einer Regeleinrichtung, und insbesondere ein Therapiegerät zur.extrakorporalen Blutreinigung.

Bei verschiedenen Therapien, wie beispielsweise bei der Hämodialyse, ist es erforderlich, den Blutdruck des Patienten laufend zu überwachen. Hierfür werden Geräte zur unblutigen (noninvasiven) Blutdruckmessung benutzt, die eine um den Arm des Patienten herumlegbare Manschette aufweisen, die aufgepumpt und dann unter Druckmessung langsam entlastet wird. Dabei werden der systolische und der diastolische Blutdruck gemessen. Um eine quasi-kontinuierliche Blutdruckmessung zu erreichen, müssen Meßvorgänge in Intervallen von wenigen min. durchgeführt werden. Bei automatischen Blutdruckmeßgeräten pumpt sich die Manschette selbsttätig auf. Während einer mehrstündigen Therapie sind somit häufige Wiederholungen des beschriebenen Meßvorganges am Patienten erforderlich.

In US-A-4,468,219 ist ein System zur Behandlung von Blut oder anderer biologischer Fluide beschrieben, bei dem eine Vorrichtung vorgesehen ist, die eine konstante Fließrate des Fluids mittels einer peristaltischen Pumpe erzeugt. Gemessen wird der Blutdruck am Saugeinlass der Pumpe. Die Flussrate wird anhand der Pumpencharakteristik bestimmt, die die Abhängigkeiten zwischen dem Eingangsdruck der Pumpe und der Blutflussrate bzw. Pumpendrehzahl angibt. Da es sich bei diesen Größen um rein technische Parameter der Pumpe handelt, sind die Pumpencharakteristiken durch mathematische Beziehungen darstellbar.

US-A-5,178,603 beschreibt ein Pumpensystem zur Regelung der Flussrate bei Veränderung des Ansaugdrucks einer Pumpe in einem extrakorporalen System zur intravenösen Blutentnahme und Reinfusion. In jeder Behandlung werden mehrfach die aktuellen Strömungsverhältnisse in der venösen Blutlinie zwischen dem Blutgefäßzugang und der ersten Pumpe ermittelt und der Ansaugdruck und die Flussrate werden automatisch gemessen. Daraus werden mittels linearer Extrapolation Lage und Neigung der Arbeitskennlinien und der Arbeitsbereich der Pumpe festgelegt. Bei Überschreiten einer Grenz-Kennlinie wird Alarm ausgelöst.

Der Erfindung liegt die Aufgabe zu Grunde, eine Therapieeinrichtung mit intervallartiger Blutdruckmessung derart auszubilden, dass die Zahl der Meßvorgänge reduziert ist.

Die Lösung dieser Aufgabe erfolgt erfindungsgemäß mit den Merkmalen des Patentanspruchs 1. Hiernach unterbleibt die Druckmessung an einigen der dafür vorgesehenen Zeitpunkten und sie wird durch von der Regeleinrichtung vorgegebene hypothetische Werte ersetzt, die aus einem früheren Blutdruckverlauf bei einem Therapievorgang (desselben Patienten) gewonnen wurden.

Klinische Erfahrungen mit Dialysepatienten haben gezeigt, dass die Blutdruckverläufe der Patienten während der Dialysebehandlung trotz der intraindividuellen Variabilität innerhalb patientenspezifischer Streubereiche liegen. Wird nach einem ersten Behandlungsabschnitt von beispielsweise 45 min. der aktuelle Blutdruckverlauf einem statistischen Vergleich mit den gespeicherten Verläufen vorangegangener Behandlungen desselben Patienten unterzogen, so kann ein vorangegangener Behandlungsverlauf als Leitkurve herangezogen werden, dessen Werte als Ist-Werte des Blutdrucks angenommen werden können. Dadurch kann ein hoher Anteil der gegenwärtig erforderlichen Blutdruckmessungen entfallen. Der aktuelle Blutdruckverlauf kann während einer Anfangsphase in kurzen Abständen von beispielsweise 5 min. gemessen und mit gespeicherten Blutdruckverläufen vorangegangener Behandlungen desselben Patienten verglichen werden. Anhand statistischer Analysen und der relevanten Behandlungsparameter wird aus einem Datenspeicher der frühere Blutdruckverlauf mit der größten Ähnlichkeit zum aktuellen Blutdruckverlauf ermittelt und für die laufende Behandlung als Leitkurve der Blutdruckregelung verwendet.

Durch die Erfindung werden patientenspezifische Erfahrungen über das Blutdruckverhalten bei vorangegangenen Therapiebehandlungen aktiv in die Optimierung der aktuellen Behandlung einbezogen. Durch das Leitkurven-Verfahren können in Abhängigkeit vom Behandlungsablauf etwa 60 % der bisher erforderlichen Blutdruckmessungen am Patienten entfallen. Das memory-basierte Verfahren erfordert keinerlei zusätzliche Meßeinrichtungen oder Geräte. Die erforderliche Rechen- und Speicherkapazität ist gering.

Vorzugsweise führt die Regeleinrichtung, die u.a. die Blutdruckmessung steuert, die folgenden Schritte aus:
a) Erstellen und Speichern einer Sammlung der zeitlichen Verläufe des Blutdrucks bei mehreren Therapievorgängen,
b) Durchführung von aufeinanderfolgenden Blutdruckmessungen während eines Therapievorganges zum Erhalten eines aktuellen Blutdruckverlaufs,
c) Ermitteln eines Blutdruckverlaufs aus den gespeicherten Verläufen, der mit dem aktuellen Blutdruckverlauf die größte Ähnlichkeit hat, als Leitkurve,
d) Regelung des Blutdrucks unter Benutzung von Werten der Leitkurve für den Ist-Wert des Blutdrucks.

Gemäß einer bevorzugten Weiterbildung der Erfindung ist vorgesehen, dass im Anschluss an die Benutzung von Werten der Leitkurve wieder eine neue Messung des Blutdrucks erfolgt und der neue Meßwert als Ist-Blutdruck für die Regelung benutzt wird. Nach der neuen Messung kann eine Überprüfung des bisherigen Blutdruckverlaufs auf Ähnlichkeit mit den gespeicherten Blutdruckverläufen durchgeführt werden, um ggf. eine neue Leitkurve auszuwählen. So kann nach jeder Blutdruckmessung die statistische Analyse wiederholt werden, um für die jeweilige Behandlungsphase die optimale Leitkurve auszuwählen.

Gemäß einer bevorzugten Weiterbildung der Erfindung ist vorgesehen, dass das Maß der Ähnlichkeit des bisherigen Blutdruckverlaufs mit einem gespeicherten Blutdruckverlauf entscheidet, wie groß das Zeitintervall ist, das bis zur nächsten Messung verstreicht. Für die Ähnlichkeitsmessung können bekannte statistische Methoden herangezogen werden, wie beispielsweise der Student's t-Test, Mustererkennungsverfahren oder Mittelwertverfahren oder Kombinationen hiervon. Diese Verfahren liefern jeweils einen Ähnlichkeitswert, der beispielsweise um so größer ist, je größer die Ähnlichkeit der miteinander verglichenen Kurven ist. Das Maß der Ähnlichkeit wird erfindungsgemäß ausgenutzt, um die Dauer des Intervalls zwischen zwei Blutdruckmessungen festzulegen.

Die Erfindung eignet sich insbesondere für Dialysegeräte und andere Geräte für die extrakorporale Blutbehandlung, jedoch auch beispielsweise für die Infusionstherapie.

Im folgenden wird unter Bezugnahme auf die Zeichnungen ein Ausführungsbeispiel der Erfindung näher erläutert.

Es zeigen:
- Fig. 1: eine schematische Darstellung eines Dialysegerätes,
- Fig. 2: ein Zeitdiagramm der Dialysebehandlung unter Darstellung der Ultrafiltrationsrate und des Blutdrucks und
- Fig. 3: ein Fuzzy-Set für die linguistische Variable "relatives Ultrafiltratvolumen UF/GE".

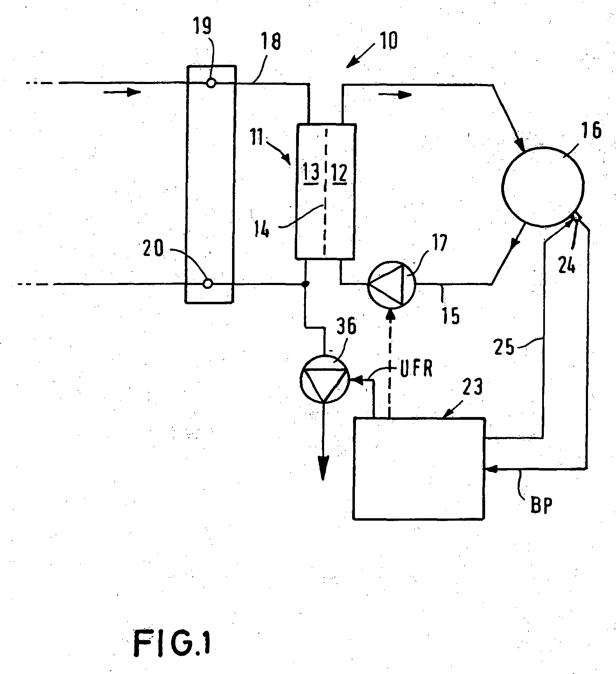

Die in Fig. 1 dargestellte Dialysemaschine entspricht in ihrem Grundaufbau derjenigen von EP 0 956 872 A2. Die Dialysemaschine 10 enthält eine Ultrafiltrationsvorrichtung 11 mit einer Primärkammer 12 und einer Sekundärkammer 13, die durch eine Membran 14 voneinander getrennt sind. Die Primärkammer 12 ist Bestandteil eines Blutkreislaufs 15, bei dem Blut, welches über das arterielle System des Patienten entnommen wurde, in der Ultrafiltrationsvorrichtung 11 gereinigt und anschließend über das venöse System zum Patienten 16 zurückgeführt wird. In dem Blutkreislauf 15 befindet sich eine Pumpe 17. Diese ist als volumetrische Pumpe ausgebildet, d.h. die Fördermenge dieser Pumpe entspricht der Antriebsgeschwindigkeit, und ist steuerbar.

Die Sekundärkammer 13 der Ultrafiltrationsvorrichtung 11 befindet sich in einem Dialysierflüssigkeitsweg 18, in dem Dialy-sierflüssigkeit gepumpt wird. Die Dialysierflüssigkeit kommt von einem (nicht dargestellten) Vorratsbehälter, nimmt in der Ultrafiltrationsvorrichtung 11 zusätzliche Substanzen aus dem Blut auf und wird dann zu einem (nicht dargestellten) Ablauf gepumpt. In dem Dialysierflüssigkeitsweg ist vor und hinter der Sekundärkammer 13 jeweils eine Durchflußkammer 19 bzw. 20 angeordnet, welche die Durchflußrate an der betreffenden Stelle regelt. Die Durchflußkammer 19 und die Durchflußkammer 20 haben die gleiche Förderrate. Über die volumengesteuerte Ultrafiltrationspumpe 36 wird die gewünschte Ultrafiltrationsmenge UFV in einer festgelegten Ultrafiltrationsrate UFR abgezogen. Das Zeitintegral über die Ultrafiltrationsrate UFR bildet das Ultrafiltratvolumen UFV, also dasjenige Flüssigkeitsvolumen, das seit Beginn der Behandlung die Membran 14 passiert hat. Die Regelung der Ultrafiltrationsrate erfolgt durch die Regeleinrichtung 23, die Steuersignale für die Förderrate der Pumpe 36 liefert. Die Förderrate der Pumpe wird in der Weise eingestellt, dass sich eine gewünschte Ultrafiltrationsrate ergibt.

Die Regeleinrichtung 23 empfängt ferner das Blutdrucksignal BP eines Blutdruckmessers 24, der am Körper des Patienten angebracht ist. Der Blutdruckmesser weist eine aufblasbare Manschette auf, die um den Oberarm des Patienten gelegt ist, und führt in Intervallen nicht-invasive Blutdruckmessungen aus. Die Steuerung der Intervalle erfolgt über Leitungen 25 durch die Regeleinrichtung 23. Neben dem Blutdruckwert BP können der Regeleinrichtung auch noch Blutdrucktrendwerte zugeführt werden, wie dies in EP 0 956 872 A2 beschrieben ist. Die Regeleinrichtung 23 regelt in Abhängigkeit von den ihr zugeführten Eingangsgrößen die Ultrafiltrationsrate UFR.

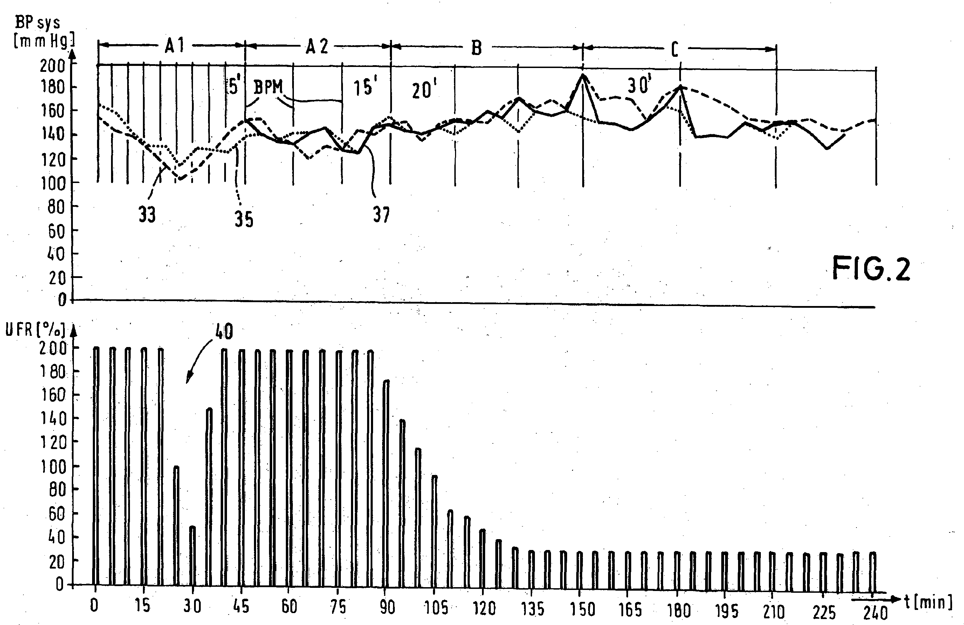

Fig. 2 zeigt in der unteren Hälfte ein Beispiel des zeitlichen Verlaufs der relativen Ultrafiltrationsrate UFR (in %). Bei einer Dialysebehandlung wird zunächst mit hoher relativer Ultrafiltrationsrate von 200 % gearbeitet und anschließend wird die Ultrafiltrationsrate auf einen Minimalwert von etwa 35 % abgesenkt.

In der oberen Hälfte von Fig. 2 ist der systolische Blutdurck BPsys eines Patienten während einer Dialysebehandlung eingetragen. Dabei zeigt die gestrichelte Linie den aktuellen Blutdruckverlauf 33, wie er sich bei kontinuierlicher Blutdruckmessung ergeben würde.

In der Regeleinrichtung 23 oder in einem zentralen Computer ist eine Sammlung aus den zeitlichen Verläufen des Blutdrucks bei früheren Therapievorgängen gespeichert.

In einer Anfangsphase A1 wird mit dem Blutdruckmeßgerät 24 in Intervallen von 5 min. der aktuelle Blutdruck gemessen und als Blutdruckverlauf 33 gespeichert. Die Blutdruckregelung erfolgt ausschließlich in Abhängigkeit von dem aktuellen Blutdruckverlauf 33. Die Anfangsphase A1 dauert bei dem vorliegenden Ausführungsbeispiel 45 min. lang. Am Ende der Anfangsphase A1 wird der aktuelle Blutdruckverlauf 33 mit den gespeicherten Blutdruckverläufen vorangegangener Behandlungen desselben Patienten verglichen. Anhand statistischer Analysen (Mittelwerte, Standardabweichungen, t-Test oder Bildverarbeitungsmethoden) wird aus dem Datenspeicher der Blutdruckverlauf mit der größten Ähnlichkeit zum aktuellen Blutdruckverlauf ermittelt und für die laufende Behandlung als Leitkurve 35 (gepunktet) benutzt.

In der sich anschließenden zweiten Phase A2 werden Blutdruckmessungen, die durch die Striche BPM bezeichnet sind, in Intervallen von 15 min. durchgeführt. In der Phase B werden die Blutdruckmessungen BPM in Intervallen von 20 min. durchgeführt und in der Endphase C in Intervallen von 30 min. Die Blutdruckregelung erfolgt jedoch weiterhin in 5-min.-Intervallen, wobei jeweils zu den Zeitpunkten, in denen keine Messung durchgeführt wird, die Werte der (gepunkteten) Leitkurve als Ist-Wert des Blutdrucks zu Grunde gelegt werden und in den Zeitpunkten der Blutdruckmessung BPM die aktuellen Werte des (gestrichelten) Blutdruckverlaufs 33. Die als Ist-Blutdruck für die Regelung benutzten Werte ergeben sich aus der durchgezogenen Kurve 37, die den "hypothetischen Blutdruckverlauf" angibt.

Aus Fig. 2 ist zu erkennen, dass zu den Zeitpunkten der Blutdruckmessung BPM der hypothetische Blutdruckverlauf, der für die Regelung maßgeblich ist, den Wert des aktuellen Blutdrucks 33 (gestrichelte Linie) annimmt, während in den dazwischenliegenden Zeitpunkten der Wert der (gepunkteten) Leitkurve angenommen wird.

Wenn der Ist-Wert unter 120 mmHg abfällt, wird die Ultrafiltrationsrate UFR verringert, so wie dies in Fig. 2 in dem Bereich 40 angegeben ist. Durch Verringerung der Ultrafiltrationsrate wird dem Patienten weniger Flüssigkeit entzogen und auf diese Weise wird der Verringerung des Blutdrucks entgegengewirkt.

Die beschriebene Verfahrensweise gewährleistet, dass die Dynamik der automatischen Anpassung der Ultrafiltrationsrate UFR und der Dialysatleitfähigkeit weitgehend erhalten bleibt.

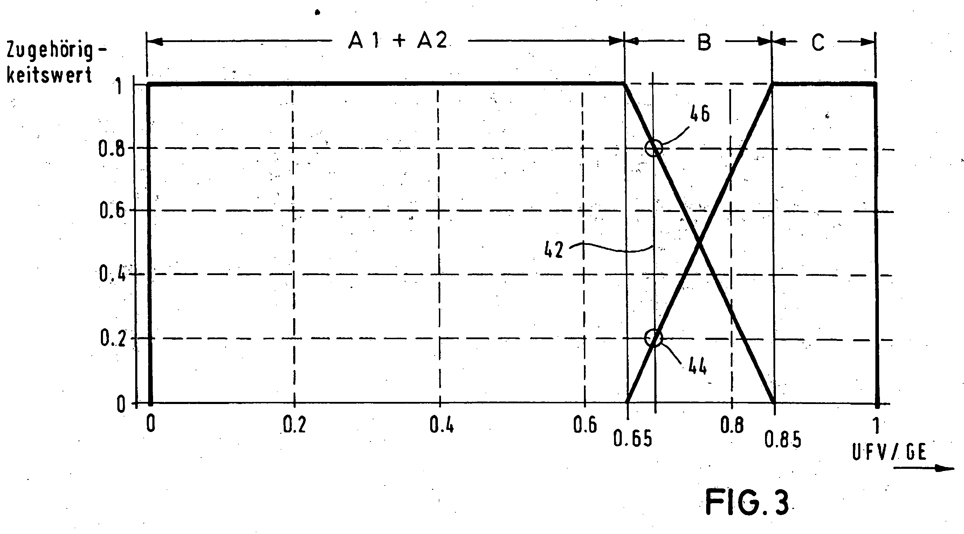

Die gesamte Behandlungsdauer wird entsprechend der in der Regeleinrichtung verwendeten Fuzzy-Sets in die Zeitabschnitte A1, A2, B und C unterteilt. Das Fuzzy-Set für die linguistische Variable "relatives Ultrafiltratvolumen UFV/GE" ist in Fig. 3 dargestellt. Hier ist längs der Abszisse das relative Ultrafiltratvolumen UFV/GE dargestellt. Hierbei ist "UFV" das gemessene Ultrafiltratvolumen und "GE" der gewünschte Gesamtentzug. Längs der Ordinate sind die Zugehörigkeitswerte von 0 bis 1 dargestellt. Für jeden Wert des aktuellen (relativen) Ultrafiltratvolumens ergibt sich eine vertikale Linie 42, deren Schnittpunkte mit den Zugehörigkeitskurven die jeweiligen Zugehörigkeitswerte 44, 46 angeben, die bei der Fuzzy-Regelung verarbeitet werden.

## Patentansprüche

1. Therapieeinrichtung mit einem am Körper eines Patienten anzubringenden, eine aufblasbare Manschette, die um den Oberarm eines Patienten legbar ist, aufweisenden Blutdruckmessgerät (24) für nicht-invasive Blutdruckmessungen und einer Regeleinrichtung (23) zur Steuerung der Zeitpunkte der Blutdruckmessung und zur Regelung des Blutdrucks in Abhängigkeit von ermittelten Blutdruckwerten,
**dadurch gekennzeichnet, dass**
die Blutdruckmessung an einigen der von der Regeleinrichtung (23) vorgegebenen Zeitpunkte unterbleibt und durch hypothetische Werte (37) ersetzt wird, die aus einem in einem Datenspeicher gespeicherten früheren Blutdruckverlauf bei einem Therapievorgang gewonnen wurden.

2. Therapieeinrichtung nach Anspruch 1, wobei die Regeleinrichtung (23) die folgenden Schritte ausführt:
a) Erstellen und Speichern einer Sammlung der zeitlichen Verläufe des Blutdrucks bei mehreren Therapievorgängen,
b) Durchführung von aufeinanderfolgenden Blutdruckmessungen (BPM) während eines Therapievorganges zum Erhalten eines aktuellen Blutdruckverlaufs (33),
c) Ermitteln desjenigen Blutdruckverlaufs aus den gespeicherten Verläufen, der mit dem aktuellen Blutdruckverlauf (33) die größte Ähnlichkeit hat, als Leitkurve (35),
d) Regelung des Blutdrucks unter Benutzung von Werten der Leitkurve (35) für den Ist Wert des Blutdrucks.

3. Therapieeinrichtung nach Anspruch 1 oder 2, wobei im Anschluss an den Schritt d) eine neue Messung des Blutdrucks erfolgt und der neue Messwert als Ist-Blutdruck für die Regelung benutzt wird.

4. Therapieeinrichtung nach Anspruch 3, wobei nach der neuen Messung eine neuerliche Überprüfung des bisherigen Blutdruckverlaufs (33) auf Ähnlichkeit mit den gespeicherten Blutdruckverläufen erfolgt.

5. Therapieeinrichtung nach einem der Ansprüche 1-4, wobei das Maß der Ähnlichkeit des bisherigen Blutdruckverlaufs (33) mit einem gespeicherten Blutdruckverlauf entscheidet, wie groß das Zeitintervall ist, das bis zur nächsten Messung verstreicht.

6. Therapieeinrichtung nach einem der Ansprüche 1-5, wobei bei Unterschreiten eines unteren Grenzwertes und/oder Überschreiten eines oberen Grenzwertes des Blutdrucks die Benutzung der Leitkurve blockiert wird.

## Claims

1. Therapy means comprising a blood pressure meter (24) comprising an inflatable cuff which can be placed around the patient's upper arm, for non-invasive blood pressure measurements and a controller (23) for controlling the times of blood pressure measurement and for controlling the blood pressure as a function of the determined blood pressure values,
**characterized in that**
the blood pressure measurement is omitted at some times provided by controller (23) and is substituted by hypothetical values (37) gathered from a former blood pressure curve determined during a therapy session which is stored in a data storage.

2. Therapy means according to claim 1, wherein the controller (23) carries out the following steps:
a) preparing and storing a collection of the time histories of the blood pressure determined during a plurality of therapy sessions,
b) carrying out successive blood pressure measurements (BPM) during a therapy session to obtain a current blood pressure curve (33),
c) determining, from the stored curves, as the basic curve (35) that blood pressure curve which shows the largest similarity to the current blood pressure curve (33),
d) controlling the blood pressure utilizing values of the basic curve (35) for the actual blood pressure value.

3. Therapy means according to claim 1 or 2, wherein subsequent to step d), another measurement of the blood pressure is carried out and the new measured value is used as the actual blood pressure for control purposes.

4. Therapy means according to claim 3, wherein after the new measurement, another check of the previous blood pressure curve (33) for similarity to the stored blood pressure curves is carried out.

5. Therapy means according to one of the claims 1-4, wherein the extent of similarity of the previous blood pressure curve (33) to a stored blood pressure curve decides the duration of the time interval up to the next measurement.

6. Therapy means according to one of the claims 1-5, wherein use of the basic curve is inhibited when a lower limit value of the blood pressure is not reached and/or an upper limit value of the blood pressure is exceeded.

## Revendications

1. Dispositif pour thérapie avec un tensiomètre (24), à placer sur le corps d'un patient, présentant un manchon gonflable qui peut être appliqué autour du segment supérieur du bras d'un patient, pour des mesures non invasives de la pression artérielle et avec un dispositif de réglage (23) pour la commande des moments de mesure de la pression artérielle et pour le réglage de la pression artérielle en fonction des valeurs de pression artérielle déterminées,
**caractérisé en ce que**
la mesure de la pression artérielle est annulée à quelques-uns des moments prédéterminés par le dispositif de réglage (23) et est remplacée par des valeurs hypothétiques (37) qui ont été obtenues à partie d'une évolution de la pression artérielle antérieure mémorisée dans une mémoire de données dans un processus thérapeutique.

2. Dispositif pour thérapie selon la revendication 1, dans lequel le dispositif de réglage (23) exécute les étapes suivantes :
a) production et mémorisation d'un recueil d'évolutions temporelles de la pression artérielle dans plusieurs processus thérapeutiques,
b) réalisation de mesures de la pression artérielle (BPM) consécutives pendant un processus thérapeutique pour obtenir une évolution actuelle de la pression artérielle (33),
c) détermination de l'évolution de la pression artérielle à partir des évolutions mémorisées, qui a la plus grande similitude avec l'évolution actuelle de la pression artérielle (33), en tant que courbe directrice (35),
d) régulation de la pression artérielle en utilisant des valeurs de la courbe directrice (35) pour la valeur réelle de la pression artérielle.

3. Dispositif pour thérapie selon la revendication 1 ou 2, dans lequel à la suite de l'étape d), une nouvelle mesure de la pression artérielle s'effectue et la nouvelle valeur de mesure est utilisée en tant que pression artérielle réelle pour le réglage.

4. Dispositif pour thérapie selon la revendication 3, dans lequel après la nouvelle mesure, une vérification de l'évolution de la pression artérielle (33) actuelle s'effectue à nouveau de manière similaire aux évolutions de la pression artérielle mémorisées.

5. Dispositif pour thérapie selon l'une des revendications 1 à 4, dans lequel la mesure de la similitude de l'évolution de la pression artérielle actuelle (33) détermine avec une évolution de la pression artérielle mémorisée, la durée de l'intervalle temporel qui s'écoule jusqu'à la prochaine mesure.

6. Dispositif pour thérapie selon l'une des revendications 1 à 5, dans lequel lors du dépassement vers le bas d'une valeur seuil inférieure et/ou du dépassement vers le haut d'une valeur seuil supérieure de la pression artérielle, l'utilisation de la courbe directrice est bloquée.
